# EUROPEAN PATENT APPLICATION

(11) **EP 1 217 078 A1**
(43) Date of publication of application: **26.06.2002**
(21) Application number: 00128176.5
(22) Date of filing: 21.12.2000
(51) Int. Cl.: C12Q 1/42, G01N 33/573

(54) **Phosphatase immunoassay using antibodies against bisphosphorylated substrates**

(71) Applicant: Evotec OAI AG, 22525 Hamburg (DE)
(72) Inventor: Krämer, Joachim, Dr., 25474 Ellerbek (DE); Peiker, Christiane, 25469 Halstenbek (DE); Henco, Karsten, Dr., 40699 Erkrath (DE)
(74) Representative: Draudt, Jutta, Dr.

(57) **Abstract**

In the present invention a process for detecting enzyme activity in an immunoassay is described, the process comprising the following steps:
a) providing a protein, peptide or a derivative thereof comprising the sequence motif

   -Z-X-Y- or -Y-X-Z-

   wherein
   Z = an amino acid to be modified by the enzyme
   X = a sequence of between 0 and 1000 amino acids which may be the same or different
   Y = an affinity enhancer for the binding to an antibody as a substrate for the enzyme;
b) incubating the protein, peptide or a derivative thereof with the enzyme to form a modified protein, peptide or a derivative thereof;
c) adding the antibody having a specificity to the peptide, protein or a derivative thereof that has been modified at the amino acid in the Z position; and
d) detecting the enzyme activity.

## Description

The present invention relates to a process for detecting enzyme activity in an immunoassay. The invention further concerns a kit for carrying out the assay and to a luminescently labelled ligand.

In eucaryotic cells there are numerous post-translational modifications of proteins which are introduced after termination of the protein synthesis. In the post-translational modification of proteins the action of corresponding modification enzymes is required. There is known a great number of post-translational modifications which are introduced after termination of the protein synthesis.

As an example the post-translational formation of disulfide bridges by the enzyme disulfide diisomerase may be mentioned.

Similarly, particular chemical groups may be added to amino acids in a protein. Representative examples are the glycosylation, phoshorylation, acetylation, acylation and carboxylation. Said enzyme-mediated reactions are generally reversible.

Detecting the activity of enzymes, for example, involved in post-translational modification, would be useful for the high-throughput screening of chemical libraries. Modulators of enzymes activity could eventually be developed into drugs used for the treatment of e. g. Parkinson's disease, cancer, or diabetis mellitus and other metabolic disorders.

In the meantime several assays for detecting enzyme activity have been developed. In a commercially available phosphatase assay, the release of phosphate from serine or threonine is detected by an absorbance change using Malachite Green as an indicator (Upstate Biotechnology, U.S.A., catalogue numbers # 14-110 and # 14-111). This assay principle is a time consuming multi-step procedure and is highly sensitive to phosphate contaminations. Therefore, the different components of the phosphatase assay have to be carefully purified prior to the experiment.

In a further conventional approach, radioactivity is used to detect dephosphorylation of phospho-serine or phospho-threonine by the activity of a phosphatase by the release of ³²P from the substrate peptide or protein (Current Protocols in Molecular Biology, Unit 18.2, John Wiley & Sons). However, there is a growing demand away from the use of radioactivity in assay applications, because of problems with costs, safety, disposal, and shelf-life of the ligand reagents.

It was therefore an object of the present invention to establish an assay method for detecting enzyme activity. The assay method should be highly reliable and simple to perform without the need to develop specific high affinity antibodies.

This object has been solved by the assay process according to the features of claim 1.

The present invention relates to a process for detecting enzyme activity in an immunoassay comprising the following steps:
a) providing a protein, peptide or a derivative thereof comprising the sequence motif

   -Z-X-Y-

   or

   -Y-X-Z-

   wherein
   Z = an amino acid to be modified by the enzyme
   X = a sequence of between 0 and 1000 amino acids which may be the same or different
   Y = an affinity enhancer for the binding to an antibody as a substrate for the enzyme;
b) incubating the protein, peptide or a derivative thereof with the enzyme to form a modified protein, peptide or a derivative thereof;
c) adding the antibody having a specificity to the peptide, protein or a derivative thereof that has been modified at the amino acid in the Z position; and
d) detecting the enzyme activity.

By detecting the presence, absence or amount of a complex between the modified protein, peptide or derivative thereof and the antibody, an enzyme activity can be measured.

The subclaims define preferred embodiments of the process of the present invention.

The accompanying figures illustrate the present invention. In the figures the following is shown:
- Figure 1: is a schematic drawing of the principle of the direct assay of the present invention.
- Figure 2: is a schematic drawing of the principle of the indirect assay of the present invention.
- Figure 3: is a schematic drawing of a preferred embodiment of the principle of the direct assay of the present invention to detect phosphatase activity.
- Figure 4: is a diagram showing the specific binding of the polyclonal anti-active JNK antibody to TAMRA-labelled P1°*-peptide.
- Figure 5: is a diagram showing the competition of the binding of the polyclonal JNK antibody to the TAMRA-labelled P1°*-peptide.
- Figure 6: is a diagram of a direct phosphatase assay (see Fig. 3).
- Figure 7: is a diagram of a direct phosphatase assay (see Fig. 3).
- Figure 8: is a diagram of a direct phosphatase assay (see Fig. 3).
- Figure 9: is a diagram showing the inhibition of the PP2A-dependent dephosphorylation of the TAMRA-P1°*-peptide substrate by the phosphatase inhibitor microcystin-LR.
- Figure 10: is a diagram showing the inhibition of the PP2A-dependent dephosphorylation of the TAMRA-P1°*-peptide substrate by the phosphatase inhibitor ocadaic acid.
- Figure 11: is a diagram of a direct phosphatase assay (see Fig. 3).
- Figure 12: is a diagram of a direct phosphatase assay (see Fig. 3).
- Figure 13: is a schematic drawing of the principle of the indirect assay of the present invention for detecting phosphatase activity.
- Figure 14: is a diagram of an indirect phosphatase assay (see Fig. 13).

In the sequence motif, any amino acid or sequence of amino acids (X) can be inserted between Z and Y. The number of amino acids is in the range of 0 to 1000 amino acids. A range of 0 to 1000 is adequate to include both linear and conformational antibody epitopes. X is particularly at least one amino acid, any other short amino acid sequences having at least two amino acids, such as oligopeptides, being also included. In an preferred embodiment, X is in a range of 1 to 10 amino acids.

The antibody used is usually a monoclonal or polyclonal antibody. It is essential that the antibody has a specificity to the peptide, protein or derivative thereof that has been modified at the amino acid in the Z position.

In the event that the protein, peptide or the derivative thereof is enzyme-modified by addition of chemical groups to Z in the sequence motif, a corresponding co-substrate is used in step (b). Suitable co-substrates, are, for example, a phosphate, mono- or oligosaccharide, biotin, an acyl group, an acetyl group, a hydroxyl group or an amide group.

The affinity enhancer Y contained in the sequence motif has the function to promote and/or to intensify the binding of an antibody which is added in step (c) of the process of the present invention. The affinity enhancer Y can comprise at least one amino acid. Depending on the type of reaction, for example an addition or elimination of chemical groups in the protein, peptide or derivative thereof by enzyme activity, the amino acid comprised in the affinity enhancer may be substituted.

Examples for suitable substitutions are groups such as a phosphate, mono- or oligo saccharide, biotin, an acyl group, a hydroxyl group or an amide group. Usually the substitutions on the Z and X groups are identical. It is, however, also possible that Z and Y are substituted differently.

Depending on the enzyme modification reaction such as the enzymatic addition or elimination of groups to or from the protein, peptide or the derivatives thereof, the enzyme may be of such kind that either the addition or the elimination of the chemical group is promoted by the enzyme. As examples, enzymes such as a kinase, phosporylase, carboxylase, decarboxylase, acylase, deacylase, hydroxylase dehydroxylase, amidase, deamidase, acetylase or deacetylase may be mentioned.

The immunoassay of the present invention may be performed as a direct binding immunoassay, preferably a homogeneous direct binding immunoassay.

A homogeneous direct binding immunoassay is based on a mix and measure principle. This is highly advantageous over the conventional direct binding assays always requiring complicated separation steps of the assay components before detection.

In the direct binding immunoassay, an optically detectable peptide, protein or derivative thereof, or an optically detectable antibody is used. The optical detection labelling may be carried out according to conventional standard techniques. Preferably, the peptide/protein/derivative thereof or antibody is labelled using a luminescent or a radioactive tag or by using specific labelling molecules such as a reporter enzyme or an affinity ligand.

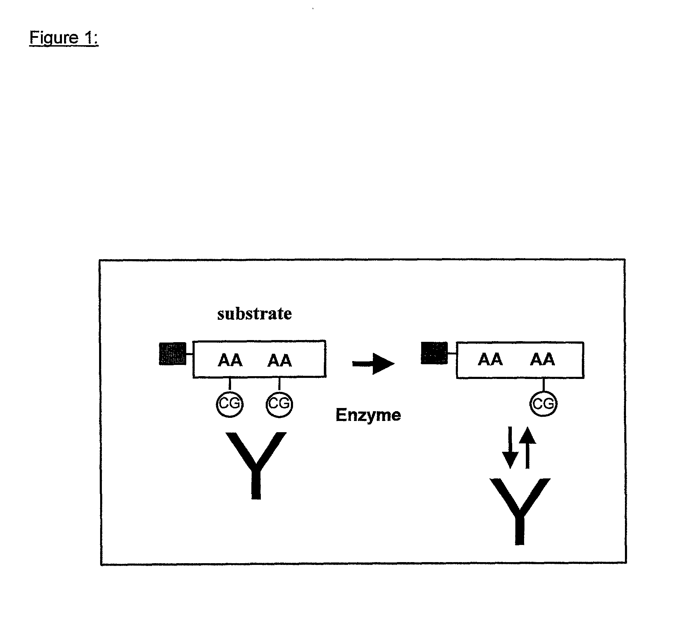

In figure 1 a schematic drawing of the principle of the direct assay of the present invention is shown. A labelled modified protein/peptide substrate carries two chemical groups (CG) on two adjacent amino acids (AA). By the action of an enzyme, one of the CGs is cleaved off. Consequently, the labelled modified substrate does no more bind to the specific antibody.

The assay of the present invention may be also be performed as a competition immunoassay, preferably a homogeneous indirect binding immunoassay. A homogeneous indirect binding immunoassay is also based on the mix and measure principle.

In this indirect binding immunoassay, an optically detectable ligand is added to compete with the modified peptide or protein for binding to the antibody. The ligand is preferably made optically detectable or labelled using a luminescent or a radioactive tag or by using specific labelling molecules such as a reporter enzyme or an affinity ligand.

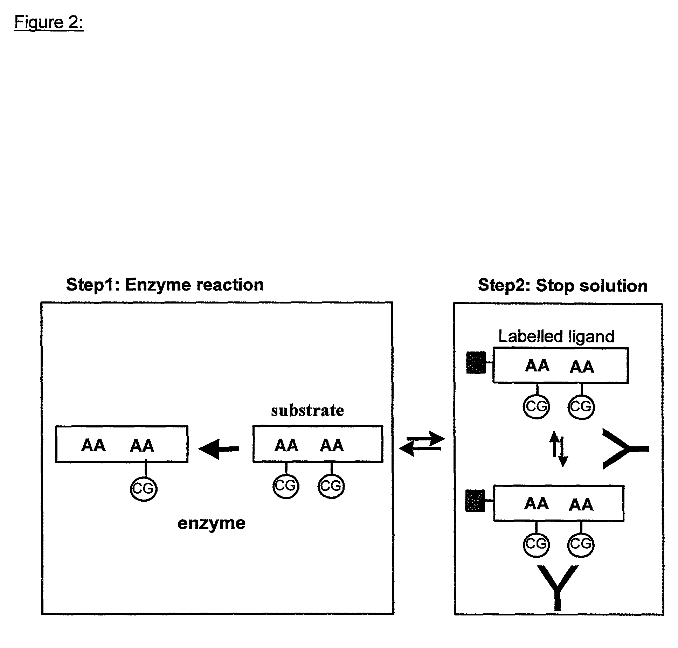

Figure 2 shows a schematic drawing of the principle of the indirect assay of the invention. In a first step, a modified protein/peptide substrate having two chemical groups (CG) on two adjacent amino acids (AA) is subjected to the action of an enzyme loosing one of the chemical groups. The product of the reaction does no more compete with the labelled modified protein/peptide (ligand) for binding to the specific antibody.

The assay of the present invention can be performed as a fluorescence immunoassay. In particular, fluorescence immunoassays such as a fluorescence polarization immunoassay, a fluorescence correlation spectroscopic assay, a fluorescence lifetime assay or a fluorescence intensity distribution assay may be mentioned.

The present invention also concerns a kit for detecting enzyme activity in an immunoassay. Said kit comprises the following components:
- an enzyme;
- a substrate as defined before;
- an antibody as defined beforehand; and
- reaction buffers including eventually a co-substrate.

In a preferred embodiment, the kit comprises a preferably luminescently labelled peptide/protein/derivative thereof ligand, said ligand comprising the following sequence motif

-Z-X-Y-

or

-Y-X-Z-

wherein
Z = an amino acid to be modified by the enzyme
X = a sequence of between 0 and 1000 amino acids which may be the same or different
Y = an affinity enhancer for the binding to the antibody.

The meanings of Z, X and Y are already described beforehand. With respect to the enzymes, antibodies, co-substrates, labelling techniques, etc., it is referred to the explanations beforehand.

The present invention also relates to a luminescently labelled ligand for an enzyme comprising the above sequence motif.

The present process as well as the present kit may be used for screening modulators for enzyme activity. Such modulators may play a key role in metabolism of animals including human beings. They are considered to be extremely useful for the treatment of metabolic disorders and cancer.

As an example, the assay of the present invention can be carried out to detect phosphatase activity. By detecting the presence, absence or amount of a complex between a bis-phosphorylated protein/peptide and an antibody phosphatase activity can be measured.

In the following a particular example of a process for detecting dephosphorylation of phospho-serine or phospho-threonine by the activity of a phosphatase will be shown. Specifically, in the assay, in the protein or peptide Z= serine or threonine and Y = tyrosine, serine or threonine, said protein or peptide being pre-phosphorylated at the Y and Z positions, a phosphatase is used to form a protein or peptide which is dephosphorylated at the Z position, the antibody has a specificity to the peptide or protein that is phosphorylated in the Y and Z positions and the activity detected is phosphatase activity.

In this exemplary assay, X in the sequence motif comprises proline or glutamate or glycine.

In case of using a protein containing the above motif, any protein containing the motif may be used. For example, a protein such as the activated JNK protein is used, which is the c-Jun N-terminal kinase, and which is also known in the literature as the stress-activated protein kinase 1 (SAPK1). For example, activated JNK1, JNK2 or JNK3 protein may be mentioned.

When it is more convenient to use a peptide substrate, the peptide sequence is preferably selected from the active-site loop, e.g. for PP1 or PP2A from the JNK1/2/3 active site. For instance, said peptide for PP1 or PP2A comprises or is composed of the amino acid sequence H-Lys-Phe-Met-Met-pThr-Pro-pTyr-Val-Val-Thr-Arg-NH₂, wherein p means phosphorylated.

When the incubation of the protein or peptide is carried out in the presence of a serine or threonine phosphatase, the phosphatase is preferably a serine/threonine protein phosphatase type 2A (PP2A) or type 2B (PP2B). In a further preferred embodiment, the incubation might be carried out using PP1, PP4 (also named PPX) or PP6 (also named PPV) as a phosphatase.

However, it is also possible to perform the assay utilising a tyrosine phosphatase with double specificity. Examples for such phosphatases comprise CL100/3CH134, PAC1, hVH-2/MKP-2, hVH-3/B23, hVH-5, MKP-3/PYST1, B59, MKP-4, and MKP-5.

It has been shown that in the embodiment a particularly preferred antibody is a polyclonal antibody. For example a polyclonal antibody specific for activated JNK. It has been revealed that the antibody specifically recognizes a phosphorylated threonine or serine residue at the Z position within both a synthetic substrate peptide or activated JNK. Such antibodies are commercially available.

A kit for detecting phosphatase activity in an immunoassay may comprise the following components:
- a phosphatase;
- a bis-phosphorylated substrate, i. e., a protein or peptide comprising the sequence motif

   Z-X-Y

   or

   -Y-X-Z

   wherein
   Z = serine or threonine
   X = a sequence of between 0 and 1000 amino acids which may be the same or different
   Y = tyrosine, serine or threonine
as a substrate for the phosphatase, said protein or peptide being pre-phosphorylated at the Y and Z positions;
- an antibody having a specificity to the peptide or protein that is phosphorylated in the Y and Z positon.

In this embodiment the kit may further comprise a detectable ligand, preferably a luminescently labelled ligand which comprises the sequence motif

-Z-X-Y-

or

-Y-X-Z-

wherein
Z = serine or threonine
X = a sequence of between 0 and 1000 amino acids which may be the same or different
Y = tyrosine, serine or threonine
wherein said protein or peptide ligand is phosphorylated at the X and Z positions.

Among the phosphatases available, the phosphatase contained in the kit of the invention may be a serine or threonine phosphatase. It is also possible to use a dual specificity tyrosine phosphatase. Examples of the serine or threonine phosphatase as well as the dual specificity tyrosine phosphatase are described above.

The phosphatase assay as well as the kit and the labelled ligand as a part of it may be used for screening for specific modulators of serine or threonine or dual specificity phosphatase activity. The assay is particularly suitable for screening compound libraries in order to locate molecules which inhibit or activate phosphatases. These molecules may be promising candidates for designing drugs used for the treatment of e.g. metabolic disorders and cancer.

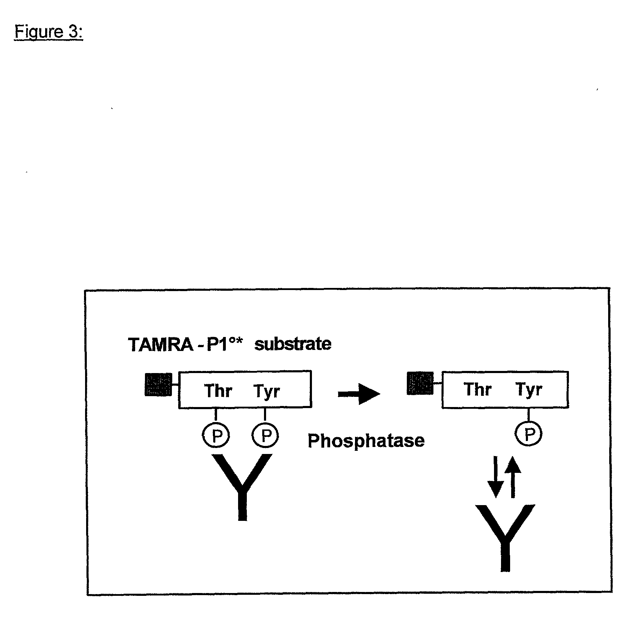

In the following, figures 3 to 14 will be explained in more detail. The abbreviations for the described peptides are used as follows: P1° H-Lys-Phe-Met-Met-Thr-Pro-pTyr-Val-Val-Thr-Arg-NH2 P1*      H-Lys-Phe-Met-Met-pThr-Pro-Tyr-Val-Val-Thr-Arg-NH2 
P1*°    H-Lys-Phe-Met-Met-pThr-Pro-pTyr-Val-Val-Thr-Arg-
         NH2
 
TAMRA-P1*°     5-TAMRA-AEEA-Lys-Phe-Met-Met-pThr-Pro-pTyr-
               Val-Val-Thr-Arg-NH2
 
TAMRA-P1°        5-TAMRA-AEEA-Lys-Phe-Met-Met-Thr-Pro-pTyr-Val-
                  Val-Thr-Arg-NH2
- TAMRA: 5'-(6-carboxytetramethylrhodamine)
- AEEA: 8-amino-3,6-dioxaoctanoic acid linker

In figure 3 a direct binding assay to detect phosphatase activity is shown. The TAMRA-P1°* substrate having the amino acid sequence 5-TAMRA-AEEA-Lys-Phe-Met-Met-pThr-Pro-pTyr-Val-Val-Thr-Arg-NH₂ (p means phosporylated) becomes dephosphorylated at the threonine residue in the presence of the phosphatase. Upon dephosphorylation, the TAMRA-labelled P1°*-peptide will no more bind to the polyclonal anti-active JNK antibody.

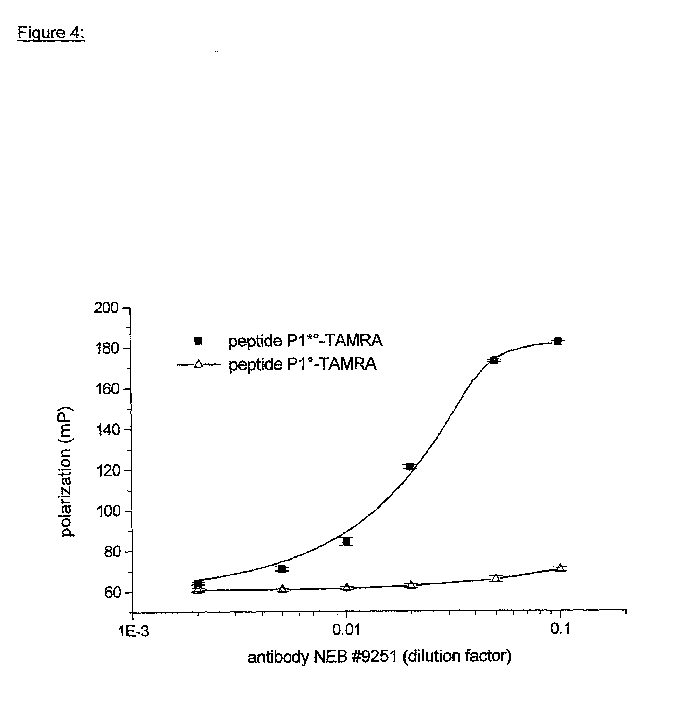

Figure 4 shows the specific binding of the polyclonal anti-active JNK antibody to TAMRA-labelled P1°*-peptide. As a control, no binding is measured for the TAMRA-labelled P1°-peptide.

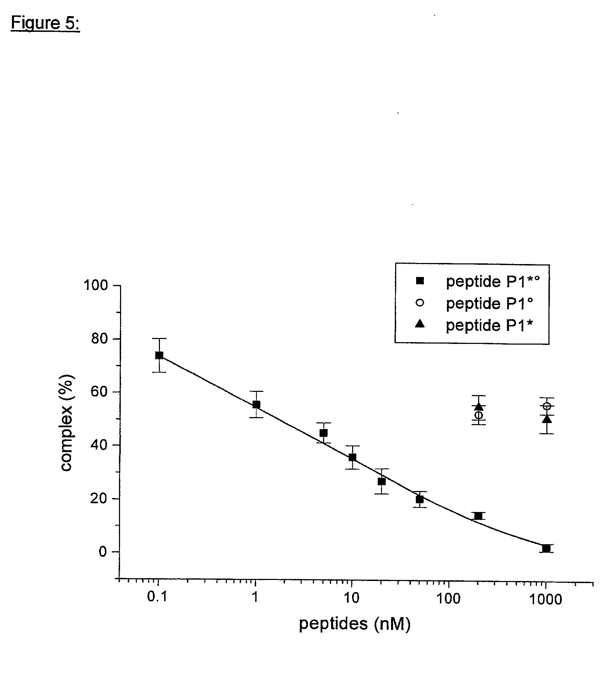

Figure 5 shows the competition of the binding of the polyclonal JNK antibody to the TAMRA-labelled P1°*-peptide using double-phosphorylated P1°*-peptide (determination of the IC50 for the P1°*-peptide competitor) and, as controls, mono-phosphorylated P1°- and P1*-peptides.

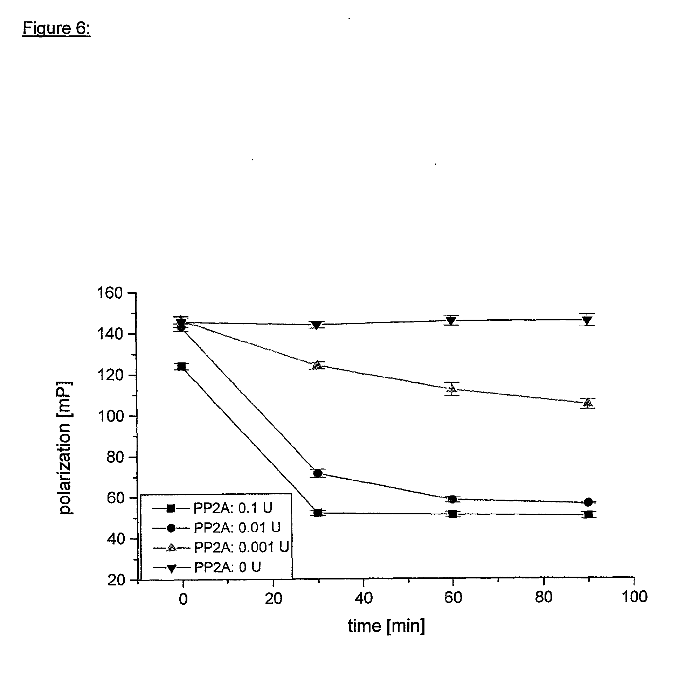

In figure 6 a diagram of a direct phosphatase assay (see Fig. 3) is given showing a time course of the dephosphorylation of the TAMRA-P1°*-peptide at different PP2A phosphatase concentrations. Desphosphorylated TAMRA-P1°*-peptide does not bind to the polyclonal anti-active JNK antibody, resulting in low fluorescence polarization.

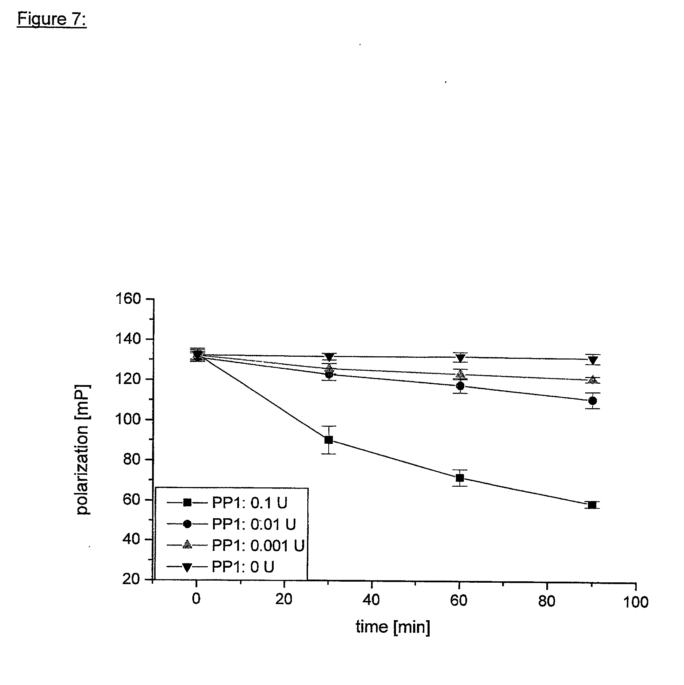

The diagram of a direct phosphatase assay (see Fig. 3) of figure 7 shows a time course of the dephosphorylation of the TAMRA-P1°*-peptide at different PP1 phosphatase concentrations. Desphosphorylated TAMRA-P1°*-peptide does not bind to the polyclonal anti-active JNK antibody, resulting in low fluorescence polarization.

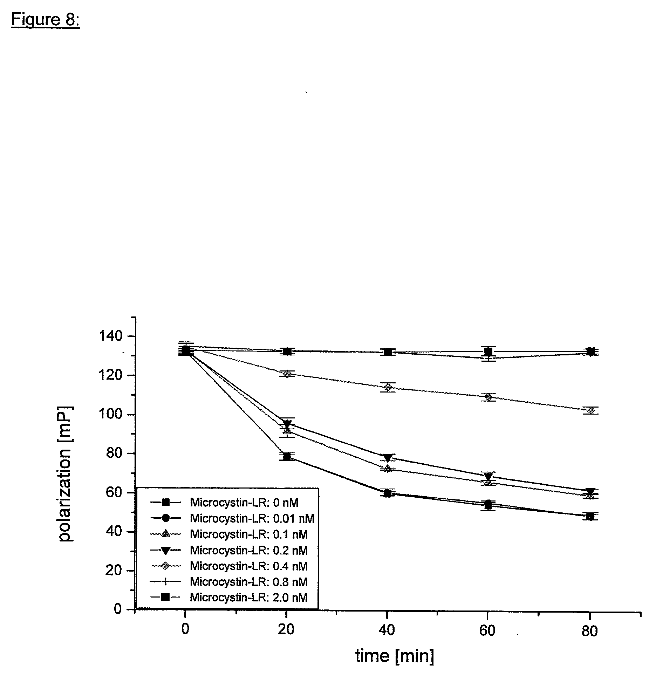

The diagram of a direct phosphatase assay (see Fig. 3) of figure 8 shows the inhibition of the dephosphorylation of the TAMRA-P1°*-peptide by PP2A phosphatase at different microcystin-LR (phosphatase inhibitor) concentrations. Desphosphorylated TAMRA-P1°*-peptide does not bind to the polyclonal anti-active JNK antibody, resulting in low fluorescence polarization.

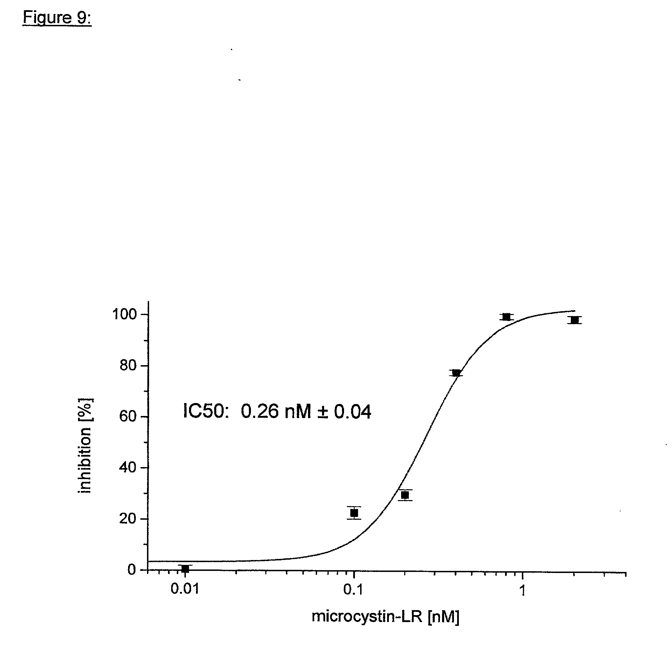

The diagram of figure 9 shows the inhibition of the PP2A-dependent dephosphorylation of the TAMRA-P1°*-peptide substrate by the phosphatase inhibitor microcystin-LR. From the inhibition curve, the half-maximal inhibition concentration (IC50) is calculated.

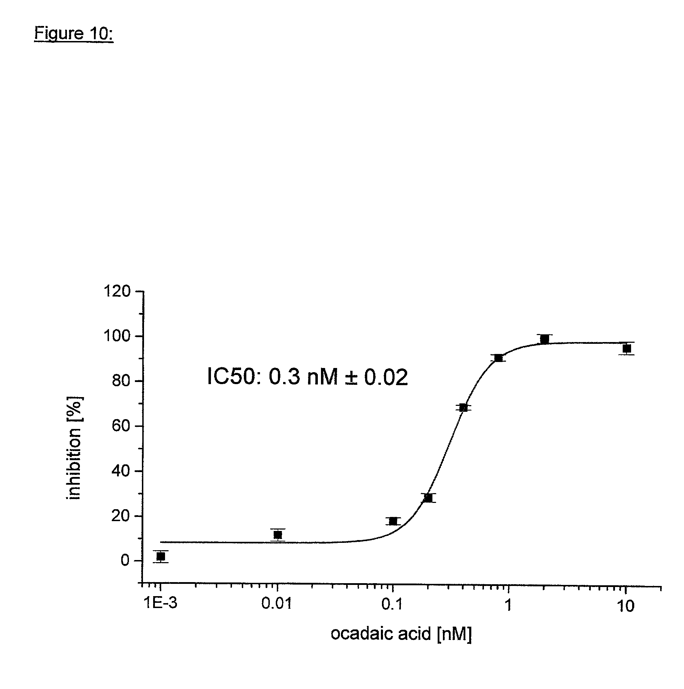

The diagram of figure 10 shows the inhibition of the PP2A-dependent dephosphorylation of the TAMRA-P1°*-peptide substrate by the phosphatase inhibitor ocadaic acid. From the inhibition curve, the half-maximal inhibitory concentration (IC50) is calculated.

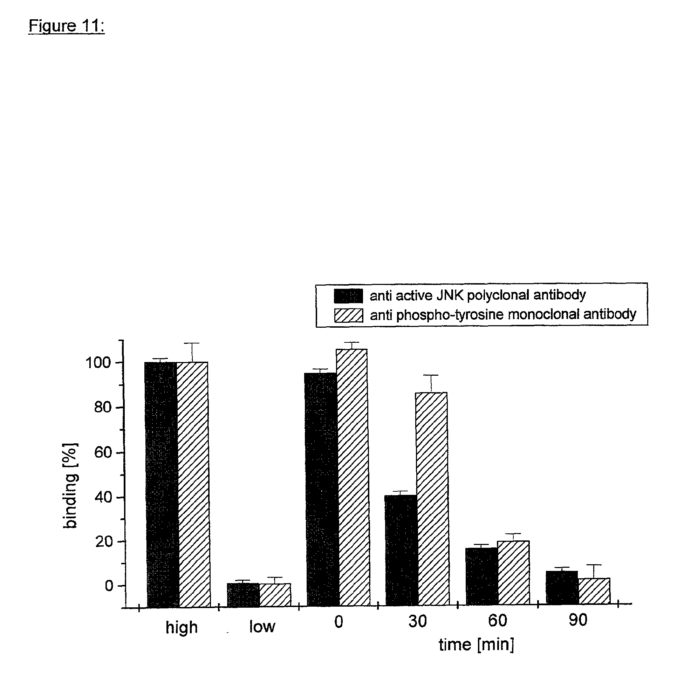

The diagram of a direct phosphatase assay (see Fig. 3) of figure 11 shows a time course of the dephosphorylation of the TAMRA-P1°*-peptide by PP1 phosphatase which is detected by two different antibodies. The polyclonal anti-active JNK antibody detects both the desphosphorylation of the phospho-threonine and/or the dephosphorylation of the phospho-tyrosine. As a control, the monoclonal anti-phospho-tyrosine antibody only detects the dephosphorylation of the phospho-tyrosine. Both events result in low fluorescence polarization. The controls denoted as "high" mean maximum binding of the antibodies to the TAMRA-P1°* peptide. The controls denoted as "low" mean no binding of the antibodies to the TAMRA-P1°* peptide due to addition of 200 nM P1°* competitor peptide.

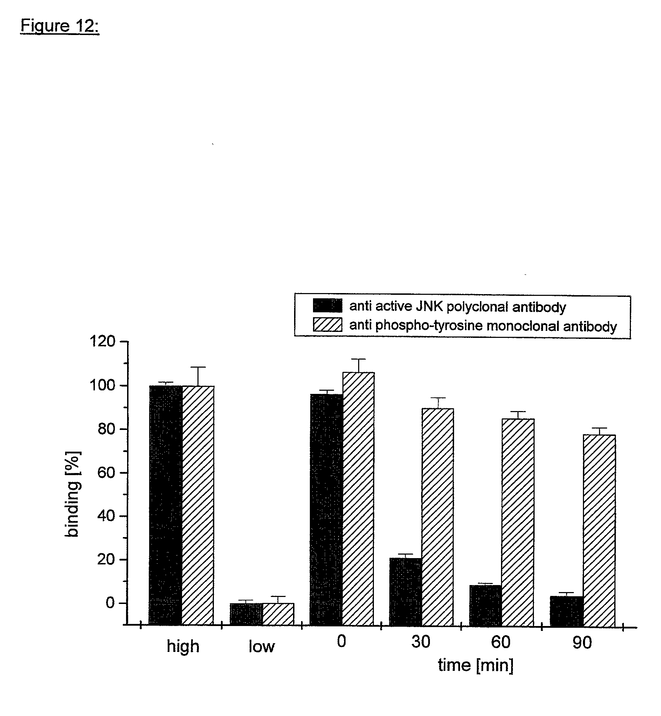

The diagram of a direct phosphatase assay (see Fig. 3) of figure 12 shows a time course of the dephosphorylation of the TAMRA-P1°*-peptide by PP2A phosphatase which is detected by two different antibodies. The polyclonal anti-active JNK antibody detects both the desphosphorylation of the phospho-threonine and/or the dephosphorylation of the phospho-tyrosine. The monoclonal anti-phospho tyrosine antibody only detects the dephosphorylation of the phospho-tyrosine. Both events result in low fluorescence polarization. The controls denoted as "high" mean maximum binding of the antibodies to the TAMRA-P1°* peptide. The controls denoted as "low" mean no binding of the antibodies to the TAMRA-P1°* peptide due to addition of 200 nM P1°* competitor peptide.

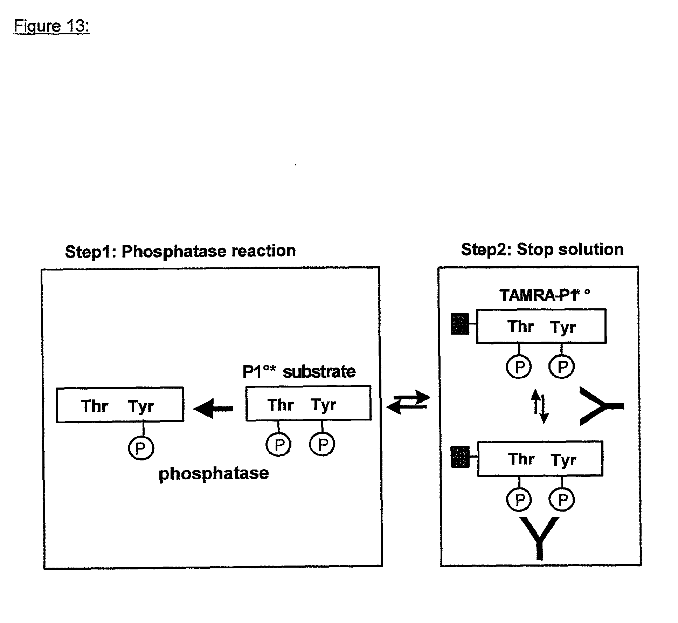

The schematic drawing of figure 13 shows the principle of the indirect assay of the present invention for detecting phosphatase activity. In a first step, the bis-phosphorylated non-fluorescent P1°*-peptide is dephosphorylated by the activity of a phosphatase. The product of the reaction (dephosphorylated P1°* peptide) will no more compete with the TAMRA-labelled P1°*-peptide for the binding to the polyclonal anti-active JNK antibody which are both added in a second step (stop solution including a reagent inactivating the phosphatase).

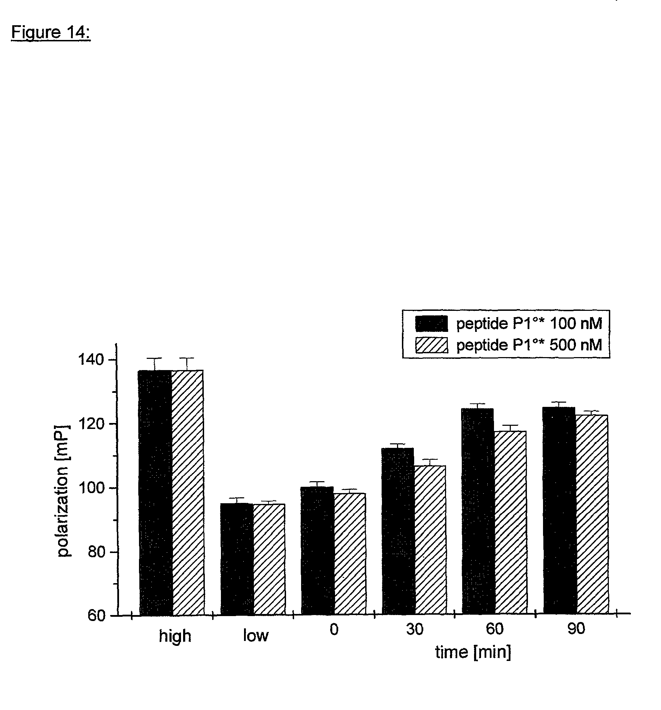

The diagram of an indirect phosphatase assay (see Fig. 13) of figure 14 shows a time course of the dephosphorylation of the P1°*-peptide substrate by PP2A phosphatase at different P1°*-peptide concentrations. Desphosphorylated P1°*-peptide does no more compete with the TAMRA-labelled P1°*-peptide for the binding to the polyclonal anti-active JNK antibody, resulting in high fluorescence polarization. The controls denoted as "high" mean maximum binding of the antibodies to the TAMRA-P1°* peptide. The controls denoted as "low" mean no binding of the antibodies to the TAMRA-P1°* peptide due to the presence of 100 nM or 500 nM P1°* substrate peptide.

The following experiments further illustrate the present invention.

### Example 1:

### Measurement of the Binding Affinity (KD) of the anti-active JNK Antibody (New England Biolabs NEB #9251)

This polyclonal anti-active antibody detects all three isoforms of the SAPK1/JNK proteins only when they are activated by dual phosphorylation at threonine₁₈₃/tyrosine₁₈₅. Binding of polyclonal anti-active JNK antibody #9251 (concentration: 0.02 mg/ml, equiv. 130 nM) to TAMRA-labelled P1*° peptide (at 5 nM) was measured at different antibody concentrations by fluorescence polarization. As a control, no binding of the antibody to the monophosphorylated TAMRA-P1° peptide (at 5 nM) is detected.

Affinity of the polyclonal anti active JNK antibody for peptide P1*°-TAMRA: K_{D} = 2.6 nM

The results of this experiment are illustrated in Fig. 4. It has been revealed that the commercially available polyclonal anti-active JNK-specific antibodies are particularly useful in the assay of the present invention. As can be seen in Fig. 4, the polyclonal anti-active JNK antibody detects the peptides - derived of the active site loop of JNK protein - only when dual phosphorylated at threonine₁₈₃ and tyrosine₁₈₅. The polarization values dramatically increase when using the double-phosphorylated TAMRA-P1*° peptide in contrast to mono-phosphorylated labelled TAMRA-P1° peptide (sequences see above).

### Example 2:

### Determination of IC50 for Peptide P1°*:

Competition of NEB #9251 antibody-P1°*-TAMRA complex with P1°* peptide was measured by fluorescence correlation spectroscopy: Binding of anti-active JNK antibody NEB #9251 (1:20 diluted) to TAMRA-labelled peptide P1*° (5 nM) was competed with non-fluorescent, double-phosphorylated peptide P1*°. Control peptides: mono-phosphorylated P1° and P1* did not compete effectively. Peptide P1*°: IC₅₀ = 5.0 nM ± 3.3 nM

The results of this experiment are illustrated in Fig.5. In Fig. 5 the binding of the same polyclonal anti-active antibody to TAMRA-labelled peptide P1*°(5 nM) in competition with the peptides P1° (mono-phosphorylated, sequence see above), peptide P1* (mono-phosphorylated, sequence see above) or peptide P1*° (double phosphorylated, sequence see above) is presented. As can be deduced from the complex formation, only bis-phosphorylated P1*° peptide is able to compete effectively with the TAMRA-labelled P1*°(peptide ligand) for binding to the antibody while the other mono-phosphorylated peptides displace less than 50% of the bound ligand even at high concentrations (up to 1 µM).

### Example 3:

### Dephosphorylation of TAMRA-P1°* Peptide by PP2A Phosphatase:

In Fig. 6 the rate of dephosphorylation of the P1°*-TAMRA substrate peptide by PP2A phosphatase is determined at various PP2A concentrations. Efficient dephosphorylation of the P1°*-TAMRA substrate peptide using the direct phosphatase assay is performed as follows: In a total volume of 80 µl the P1°*-TAMRA substrate peptide (at 10 nM) is dephosphorylated using 0.1, 0.01, or 0,001 units of PP2A. The phosphatase reactions are incubated at 30°C and aliquots (20 µl) are withdrawn after 0 min, 30 min, 60 min and 90 min and mixed with 10 µl of a stop solution containing the following reagents (all final concentrations): hydrogenperoxide 10 mM, polyclonal anti-active JNK antibody from NEB at a dilution of 1:20.

The formed dephosphorylated P1°*-TAMRA peptide product can be detected as it does not bind to the polyclonal anti-active JNK antibody. As a results, a drop of the fluorescence polarization is detected which is inversely proportional to PP2A phosphatase activity.

### Example 4:

### Dephosphorylation of TAMRA-P1°* Peptide by PP1 Phosphatase:

In Fig. 7 the rate of dephosphorylation of the P1°*-TAMRA substrate peptide by PP1 phosphatase is detected at various PP1 concentrations using the same direct assay as described in example 3. Efficient dephosphorylation of the P1°*-TAMRA substrate peptide using the direct phosphatase assay is performed as follows: In a total volume of 80 µl the P1°*-TAMRA substrate peptide (at 10 nM) is dephosphorylated using 0.1, 0.01, or 0,001 units of PP1. The phosphatase reactions are incubated at 30°C and aliquots (20 µl) are withdrawn after 0 min, 30 min, 60 min and 90 min and mixed with 10 µl of a stop solution containing the following reagents (all final concentrations): hydrogenperoxide 10 mM, polyclonal anti-active JNK antibody from NEB (1:20 dilution). The formed dephosphorylated P1°*-TAMRA peptide product can be detected as it does not bind to the polyclonal anti-active JNK antibody. As a results, a drop of the fluorescence polarization is detected which is inversely proportional to PP1 phosphatase activity.

### Example 5:

### Inhibition of the PP2A Phosphatase-dependent Dephosphorylation of TAMRA-P1°* Peptide by Microcystin-LR:

In Fig. 8 the inhibition of the dephosphorylation of P1°*-TAMRA substrate peptide by PP2A phosphatase is determined for the phosphatase inhibitor microcystin-LR. The inhibition of PP2A phosphatase is detected using the same direct assay as described in example 3. As a result, no reduction of the binding of the TAMRA-labelled P1*° peptide to the polyclonal anti-active JNK antibody is detected at high microcystin-LR concentrations due to full inhibition of PP2A.

Assay conditions: PP2A phosphatase (0.004 units) is incubated with P1°*-TAMRA peptide (10 nM) and microcystin-LR (0, 0.01, 0.1, 0.2, 0.4, 0.8, 2 nM) in a total volume of 100 µl at 30°C. At different time points aliquots are withdrawn from the enzyme reaction and mixed with hydrogen peroxide (10 nM), polyclonal anti-active JNK antibody from NEB #9251 (1:20 dilution).

### Example 6:

### Inhibition of the PP2A Phosphatase-dependent Dephosphorylation of TAMRA-P1°* Peptide by Microcystin-LR:

Fig. 9 shows the inhibition of the dephosphorylation of P1°*-TAMRA substrate peptide by PP2A phosphatase using the phosphatase inhibitor microcystin-LR. The half-maximal inhibitory concentration (IC50) for microcystin-LR is calculated from the data of Fig. 6: IC50 = 0.26 nM +/-0.04 nM.

### Example 7:

### Inhibition of the PP2A Phosphatase-dependent Dephosphorylation of TAMRA-P1°* Peptide by Ocadaic Acid:

Fig. 10 shows the inhibition of the dephosphorylation of P1°*-TAMRA substrate peptide by PP2A phosphatase using the phosphatase inhibitor ocadaic acid. The half-maximal inhibitory concentration (IC50) for ocadaic is calculated. IC50 = 0.3 nM +/- 0.02 nM.

Assay conditions: PP2A phosphatase (0.003 units) is incubated with P1°*-TAMRA peptide (10 nM) and ocadaic acid (0, 0.001, 0.01, 0.1, 0.2, 0.4, 0.8, 2, 10 nM) at 30°C. At different time points aliquots are withdrawn from the enzyme reaction and mixed with hydrogen peroxide (10 nM), polyclonal anti-active JNK antibody from NEB #9251 (1:20 dilution).

### Example 8:

### Measurement of the Specificity of PP1 phosphatase using two different Antibodies:

Fig. 11 shows the detection of PP1 phosphatase-dependent dephosphorylation of TAMRA-P1°* Peptide using the polyclonal anti-active JNK antibody (NEB #9251) and the monoclonal anti phospho-tyrosine antibody (NEB #9411). The dephosphorylation of the TAMRA-P1°* peptide by PP1 phosphatase is performed using the same direct assay as described in Fig 7, with the only exception that two antibodies with different specificities were used for the read-out.

As a result, a PP1 phosphatase-dependent dephosphorylation of the phospho-threonine and the phospho-tyrosine is detected. PP1 has a lower specificity when compared to PP2A (see example 9).

Assay conditions: PP1 phosphatase (0.1 units) is incubated with P1°*-TAMRA peptide (10 nM) at 30°C. At different time points aliquots are withdrawn from the enzyme reaction and mixed with the stop solution containing hydrogen peroxide (10 nM) along with either the polyclonal anti-active JNK antibody from NEB #9251 (1:20 dilution) or the monoclonal anti phospho-tyrosine antibody from NEB #9411 (1:100 dilution).

### Example 9:

### Measurement of the Specificity of PP2A phosphatase using two different Antibodies:

Fig. 12 shows the detection of PP2A phosphatase-dependent dephosphorylation of TAMRA-P1°* Peptide using the polyclonal anti active JNK antibody NEB #9251 and the monoclonal anti phospho-tyrosine antibody NEB #9411. The dephosphorylation of the TAMRA-P1°* peptide by PP2A phosphatase is performed using the same direct assay as described in Fig 7, with the only exception that two antibodies with different specificities were used for the read-out. As a result, a PP2A phosphatase-dependent dephosphorylation of the phospho-threonine is detected. PP2A has a high specificity for phospho-threonine.

Assay conditions: PP2A phosphatase (0.006 units) is incubated with P1°*-TAMRA peptide (10 nM) at 30°C. At different time points aliquots are withdrawn from the enzyme reaction and mixed with the stop solution containing hydrogen peroxide (10 nM) along with either the polyclonal anti active JNK antibody from NEB #9251 (1:20 dilution) or the monoclonal anti phospho-tyrosine antibody from NEB #9411 (1:100 dilution)

### Example 10:

### Dephosphorylation of non-fluorescent P1°* Peptide by PP2A Phosphatase (indirect Phosphatase Assay, see Fig. 13):

Fig. 14 shows the detection of PP2A phosphatase-dependent dephosphorylation of the P1°* substrate peptide in an indirect phosphatase assay.

In a first step (enzyme reaction), the bisphosphorylated P1°* substrate peptide is incubated with PP2A. In a second step (stop solution, including hydrogenperoxide), the polyclonal anti active JNK antibody (NEB #9251) is added together with TAMAR-P1°* peptide. As a result of the PP2A activity, the P1°* substrate peptide is dephosphorylated and does no longer compete with the TAMRA-P1°* peptide for the binding to the polyclonal anti active JNK antibody. This can be detected by an increase in fluorescence polarization.

Assay conditions: PP2A phosphatase (0.5 units) is incubated with 100 nM or 500 nM P1°* substrate peptide in a total volume of 80 µl at 30°C. At different time points, aliquots (20 µl) are withdrawn from the enzyme reaction and mixed with 10 µl of the stop solution containing hydrogen peroxide (10 nM), TAMRA-P1°* peptide at (5 nM) together with the polyclonal anti active JNK antibody from NEB (1:20 dilution).

### List of reagents of the described phosphatase assays:

### List of used peptides:

TAMRA-P1*° | 5-TAMRA-AEEA-Lys-Phe-Met-Met-pThr-Pro-pTyr-
Val-Val-Thr-Arg-NH2

TAMRA-P1°	5-TAMRA-AEEA-Lys-Phe-Met-Met-Thr-Pro-pTyr-Val-
Val-Thr-Arg-NH2

P1*°    H-Lys-Phe-Met-Met-pThr-Pro-pTyr-Val-Val-Thr-Arg-
NH2

P1° H-Lys-Phe-Met-Met-Thr-Pro-pTyr-Val-Val-Thr-Arg-NH2

P1*        H-Lys-Phe-Met-Met-pThr-Pro-Tyr-Val-Val-Thr-Arg-NH2

### Bisphosphorylated substrate peptide:

TAMRA-P1*°-Peptide 5-TAMRA-AEEA-Lys-Phe-Met-Met-pThr-Pro-pTyr-
Val-Val-Thr-Arg-NH2

Supplier: EVOTEC inhouse solid-phase peptide synthesis
HK-03-65-P1-13; M = 2089 g/mol; MALDI (2092.18)
1mM stock solution in 100% DMSO

### Bisphosphorylated competitor peptide:

P1*°-Peptide:  H-Lys-Phe-Met-Met-pThr-Pro-pTyr-Val-Val-Thr-
Arg-NH2

Supplier: EVOTEC inhouse solid-phase peptide synthesis
HK-03-60-P1-7; M = 1532 g/mol; MALDI (1531.88)
10mM stock solution in 100% DMSO

### Monophosphorylated control peptides:

P1°-Peptide: H-Lys-Phe-Met-Met-Thr-Pro-pTyr-Val-Val-Thr-
Arg-NH2

Supplier: EVOTEC in house solid-phase peptide synthesis HK-03-58-HF; M = 1451 g/mol; MALDI (1453.57)
10mM stock solution in 100% DMSO 
P1*-Peptide: H-Lys-Phe-Met-Met-pThr-Pro-Tyr-Val-Val-Thr-
Arg-NH2
 Supplier: EVOTEC in house solid-phase peptide synthesis
*HK-03-33-HF; M = 1452 g*/*mol; MALDI (1452.23)*
10mM stock solution in 100% DMSO

### Enzyme:

### PP1 phosphatase:

Supplier: Upstate Biotech cat. no. 14-110, lot no. 18524

### PP2A phosphatase:

Supplier: Upstate Biotech cat. no. 14-111, lot no. 20574.

### Anti active JNK-specific antibody:

New England Biolabs, U.S.A.: NEB #9251

### Anti phospho-tyrosine-specific monoclonal antibody pY100:

New England Biolabs, U.S.A.: NEB #9411

### Reagents and buffers:

### Assay buffer:

*10x HEPES-Assay-Buffer pH 7.2*
*500mM HEPES*
*100mM MgCl*_{*2*}
*10mM DTT*

Dissolve 77mg DTT (FLUKA cat. no. 43815; MW 154.25g/mol) [final concentration 10mM] in 30 ml of Millipore water, add 5ml of 1M MgCl₂ [final concentration 100mM], and 5.96g HEPES (FLUKA cat. no. 54457) [final concentration 500mM] and adjust pH with 1M NaOH to pH 7.2. Top up to 50 ml with Millipore water. The buffer will be filtered sterile (0.22µm) aliquoted to 1.5ml and should be stored at -20°C.

### 1M MgCl₂ :

Dissolve 10.2 g MgCl₂.6H₂O (FLUKA cat. no. 63068) in 50 ml of Millipore water. Buffer should be filtered (0.42µm) and can be stored at 4°C for several months.

### 1% (w/v) Pluronic F-127

Dissolve 0.5 g Pluronic (Sigma, cat. no. P-2443) in 50 ml of Millipore water. Stir gently to get a clear solution. Solution should be filtered (0.42µm) and can be stored at 4°C for several months.

### 1x HEPES-Assay-Buffer/0.1% Pluronic/ 0.01% BSA pH 7.5: (2ml):

*50mM HEPES*
*10mM MgCl*_{*2*}
*1mM DTT*
*0.1% Pluronic*
*0.01% BSA*

Add 50 µl 4% (w/v) Pluronic F-127 in water, add 200 µl of 10x HEPES-Assay-Buffer pH 7.5 and add 20 µl of 1% BSA in water (Merck Albumin Fraktion V) to 2 ml of Millipore water.

### 1%(w/v) Albumin Fraktion V (BSA)

Dissolve 0.1g Albumin Fraktion V (Merck cat.no.1.12018.0100) in 10ml of steril filtered (0.22µm) Millipore water and aliquote to 100µl. The stock should be stored at -20°C.

### DMSO: 100% DMSO

The solvent was purchased from SIGMA (cat. No. P-2650), sterile filtered.

### Hydrogenperoxide 30% (H₂O₂)

*Add 10µl of 30%* H₂O₂ (Merck cat.no.8.22287.1000) in 78 µl 1x HEPES-Assay-Buffer/0.1% Pluronic/ 0.01% BSA pH 7.5 (final concentration 1M).

### Microcystin-LR:

Supplier: Sigma cat.no. M-2912, lot no. 48H1100

### Ocadaic Acid:

Supplier: Sigma cat.no. 0-7760, lot no. 129H1199

## Claims

1. A process for detecting enzyme activity in an immunoassay comprising the following steps:
a) providing a protein, peptide or a derivative thereof comprising the sequence motif
-Z-X-Y-
or
-Y-X-Z-
wherein
Z = an amino acid to be modified by the enzyme
X = a sequence of between 0 and 1000 amino acids which may be the same or different
Y = an affinity enhancer for the binding to an antibody as a substrate for the enzyme;
b) incubating the protein, peptide or a derivative thereof with the enzyme to form a modified protein, peptide or a derivative thereof;
c) adding the antibody having a specificity to the peptide, protein or a derivative thereof that has been modified at the amino acid in the Z position; and
d) detecting the enzyme activity.

2. The process according to claim 1, wherein a co-substrate is used in step (b).

3. The process according to claim 1 or 2, wherein the affinity enhancer is at least one amino acid which may be substituted.

4. The process according to claim 3, wherein the amino acid is substituted by a phosphate, mono- or oligosaccharide, biotin, acetyl group, acyl group, hydroxyl group or amide group.

5. The process according to at least one of claims 1 to 4, wherein X is the range of 1 to 10 amino acids.

6. The process according to claim 5, wherein X = 1.

7. The process according to at least one of claims 1 to 6, wherein the enzyme is a kinase, phosphatase, carboxylase, decarboxylase, acylase, deacylase, hydroxylase, dehydroxylase, amidase, deamidase, acetylase or deacetylase.

8. The process according to at least one of the claims 1 to 7, wherein the immunoassay is performed as a direct binding immunoassay, preferably a homogeneous direct binding immunoassay.

9. The process according to claim 8, wherein an optically detectable peptide, protein or a derivative thereof is used.

10. The process according claim 8, wherein an optically detectable antibody is used.

11. The process according to claim 9 or 10, wherein the peptide/protein/derivative thereof or antibody is made optically detectable by a luminescent tag, a radioactive marker, a reporter enzyme or an affinity ligand.

12. The process according to at least one of the claims 1 to 7, wherein the immunoassay is performed as an indirect binding immunoassay, preferably a homogeneous indirect binding immunoassay.

13. The process according to claim 12, wherein an optically detectable modified ligand is added to compete with the modified peptide, protein or a derivative thereof for binding to the antibody.

14. The process according to claim 13, wherein the modified ligand is labelled by a luminescent tag, a radioactive marker, a reporter enzyme or an affinity enhancer.

15. The process according to at least one of claims 1 to 14, wherein the assay is performed as a fluorescence immunoassay, in particular a fluorescence polarization immunoassay, a fluorescence correlation spectroscopic assay, a fluorescence lifetime assay or a fluorescence intensity distribution assay.

16. The process according to at least one of claims 1 to 15, wherein the antibody is a monoclonal or polyclonal antibody.

17. The process according to claim 16, wherein the antibody is a polyclonal antibody.

18. A kit for detecting enzyme activity in an immunoassay comprising the following components:
- an enzyme;
- a substrate as defined in claim 1;
- an antibody as defined in claim 1; and
- reaction buffers including eventually a co-substrate.

19. A kit according to claim 18, further comprising a preferably luminescently labelled peptide/protein/derivative thereof ligand, said ligand comprising the following sequence motif
-Z-X-Y-
or
-Y-X-Z-
wherein
Z = an amino acid to be modified by the enzyme
X = a sequence of between 0 and 1000 amino acids which may be the same or different
Y = an affinity enhancer for the binding to the antibody.

20. A use of the assay process according to at least one of the claims 1 to 17 or the kit according to claim 18 or 19 for screening modulators for enzyme activity.

21. A luminescently labelled ligand for an enzyme comprising the sequence motif according to claim 19.

22. The process according to at least one of claims 1 to 17 for detecting phosphatase activity, wherein in the protein or peptide comprising a sequence motif -Z-X-Y- or -Y-X-Z- Z= serine or threonine and Y = tyrosine, serine or threonine, said protein or peptide being pre-phosphorylated at the Y and Z positions, a phosphatase is used to form a protein or peptide which is dephosphorylated at the Z position, the antibody has a specificity to the peptide or protein that is phosphorylated in the Y and Z positions and the activity detected is phosphatase activity.

23. The process according to claim 22, wherein X in the sequence motif comprises proline or glutamate or glycine.

24. The process according to claim 22 or 23, wherein the protein provided is activated JNK1, JNK2 or JNK3 protein.

25. The process according to claim 22 or 23, wherein the peptide provided includes sequences identical to those of the activated JNK1, JNK2 or JNK3 active-site loop.

26. The process according to claims 22 to 25, wherein the peptide comprises the amino acid sequence H-Lys-Phe-Met-Met-pThr-Pro-pTyr-Val-Val-Thr-Arg-NH₂ (p means phosphorylated).

27. The process according to at least one of claims 22 to 25, wherein the phosphatase is a serine or threonine phosphatase.

28. The process according to claim 27, wherein the incubation of the protein or peptide is carried out in the presence of a serine/threonine protein phosphatase type 2A (PP2A) or type 2B (PP2B).

29. The process according to claim 27 wherein the incubation of the protein or peptide is carried out in the presence of the phosphatase PP1.

30. The process according to claim 27, wherein the incubation of the protein or petide is carried out in the presence of the phosphatase PP4 (also named PPX) or PP6 (also named PPV).

31. The process according to at least one of claims 22 to 26, wherein the phosphatase is a tyrosin phosphatase with dual specificity.

32. The process according to claim 31, wherein the dual specificity phosphatase is CL100/3CH134, or PAC1, or hVH-2/MKP-2, or hVH-3/B23 or hVH-5, or MKP-3/PYST1, or B59, or MKP-4 or MKP-5.

33. The process according to at least one of the preceding claims, wherein the protein or peptide is dephosphorylated at the Y position.

34. The process according to at least one of the preceding claims, wherein the antibody is a polyclonal antibody specific for active JNK.

35. A kit for detecting phosphatase activity in an immunoassay comprising the following components
- a phosphatase;
- a substrate as defined in claim 22;
- an antibody as defined in claim 22.

36. The kit according to claim 35 further comprising a preferably luminescently labelled ligand, said ligand comprising the following sequence motif
-Z-X-Y-
or
-Y-X-Z-
wherein
Z = serine or threonine
X = a sequence of between 0 and 1000 amino acids which may be the same or different
Y = tyrosine, serine or threonine
said protein or peptide ligand being phosphorylated at the Y and Z positions.

37. The kit according to claim 35 or 36 wherein the phosphatase is a serine or threonine phosphatase.

38. A kit according to claim 35 or 36, wherein the phosphatase is a dual specificity tyrosine phosphatase.

39. A use of the assay process according to at least one of claims 22 to 34 or the kit according to at least one of the claims 35 to 38 for screening modulators for phosphatase activity, in particular inhibitors for a serine or threonine or dual specificity phosphatase.

40. A luminescently labelled ligand for a phosphatase comprising the sequence motif according to claim 36.
